# EUROPEAN PATENT SPECIFICATION

(11) **EP 0 602 565 B1**
(45) Date of publication and mention of the grant of the patent: **11.09.1996**
(21) Application number: 93119973.1
(22) Date of filing: 10.12.1993
(51) Int. Cl.: C07C 11/02, C07C 2/88

(54) **Preparation of linear alpha-olefins by a modified chain growth process**
Verfahren zur Herstellung von linearen alpha-Olefinen mittels einer geänderten Kettenwachsverfahren
Procédé pour la préparation d'alpha-oléfines linéaires au moyen d'une réaction de croissance de chaîne modifiée

(30) Priority: 14.12.1992 US 990230
(43) Date of publication of application: 22.06.1994
(73) Proprietor: ALBEMARLE CORPORATION, Richmond, Virginia 23219 (US)
(72) Inventor: Lin, Ronny W., Baton Rouge, Louisiana 70816 (US); Allen, Robert H., Baton Rouge, Louisiana 70808 (US); Hu, John N., Baton Rouge, Louisiana 70810 (US); Overstreet, Andrew D., Baton Rouge, Louisiana 70806 (US); Smith, Daniel K., Baton Rouge, Louisiana 70815 (US)
(74) Representative: Sandmair, Kurt, Dr. Dr.

(56) References cited:
- FR-A- 1 595 274
- GB-A- 773 536
- US-A- 4 918 254

## Description

This process relates generally to the preparation of linear alpha-olefins by ethylene chain growth and more particularly to an improved ethylene chain growth process for producing linear alpha-olefin products of high purity which are enriched in selected carbon numbers.

The preparation of linear alpha-olefins having from about 4 to 20+ carbon atoms by the so-called Ziegler chain growth process, which involves the reaction of a trialkylaluminum, such as triethylaluminum, with ethylene so as to add ethylene units to the alkyl groups and form a new trialkylaluminum product having a Poisson alkyl distribution, is a well known commercial process. The alpha-olefins are recovered and the trialkylaluminum is regenerated in a thermal displacement reaction using, for example, ethylene or higher olefins to displace the alpha-olefins from the trialkylaluminum chain growth-product.

GB-A-773 536 pertains to a process for the catalytic polymerisation of ethylene to form butene, hexene or higher liquid or solid parafine - like polymeres or mixtures thereof in the presence of an aluminium trialkyle which is activated by Ni, Co or Pt. The reaction proceeds by chain growth and displacement. Ni, Co or Pt are considered to accelerate the displacement reaction.

A limitation of a Poisson distribution is that when attempting to produce mainly olefin with carbon numbers of 6 to 12, then the quantity of C₄ product produced often exceeds market requirements. Also, a problem associated with the thermal displacement reaction is the formation of impurities, such as internal olefins and vinylidene olefins, due to isomerization or dimerization.

We have now provided an efficient process by which high purity linear alpha-olefin products which are enriched in selected, commercially desirable carbon numbers can be prepared.

In accordance with this invention there is provided a process for making a linear alpha-olefin product which is enriched in one or more olefins of a selected carbon number ranging from 5 to 12 which process comprises:
(a) feeding ethylene and trialkylaluminum to a chain growth reaction zone, said trialkylaluminum consisting essentially of one or more compounds represented by the formula AlR₃, in which a major portion of the R groups, which can be the same or different, contain a number of carbon atoms which is two less than a selected carbon number,
(b) reacting said ethylene and said trialkylaluminum compound in said reaction zone so as to form a chain growth product in which the chain length of the R groups is increased by an average amount such that said product is enriched in alkyl groups having the selected carbon number,
(c) feeding said chain growth product, a displacement catalyst and displacing linear alpha-olefin, a major portion of which linear alpha-olefin contains a number of carbon atoms which is two less than a selected carbon number, to a displacement zone so as to form said linear alpha-olefin product and trialkyl-aluminum displacement product,
(d) after the displacement reaction has proceed to the desired extent but before any significant isomerization of the linear alpha-olefin has occured, deactivating said displacement catalyst with a deactivating amount of a catalyst poison selected from lead, copper and compounds thereof, and
(e) recycling said trialkylaluminum displacement product to said chain growth reaction zone.

The aluminum alkyl feedstock for the chain growth reaction includes one or more trialkylaluminum compounds represented by the formula AlR₃ where a major portion, preferably at least 60%, and more preferably at least about 90%, of the R groups, which can be the same or different, contain at least 3 and preferably from 4 to 10 carbon atoms. The aluminum alkyl feed is chosen so as to contain R groups which have 2 less carbon atoms than the alpha-olefin or olefins which it is desired to preferentially produce. For example, if a product enriched in 1-hexene is desired, then tri-n-butylaluminum is chosen as the feedstock. Similarly if a product enriched in 1-decene is desired, then tri-n-octylaluminum is used as the feedstock. Also, if a product enriched in more than one olefin such as 1-hexene and 1-decene is desired, then either a mixed feed of tri-n-butyl aluminum and tri-n-octyl aluminum or a trialkylaluminum containing both n-butyl and n-octyl groups is used. Other combinations can be used, including trialkylaluminum compounds containing both even and odd numbers of carbon atoms such as n-propyl and n-butyl.

The chain growth reaction can be carried out batchwise or continuously by feeding ethylene and trialkylaluminum feed to a reactor zone such as an autoclave, a continuous stirred tank reactor or a tubular reactor.

Suitable ethylene pressures for the chain growth reaction generally range from 6.9-34.5 MPa (1000 to 5000 psig), preferably 13.8-24.1 MPa (2000 to 3500 psig), and reaction zone temperatures of from 100 to 250°C, preferably 120 to 160°C. The reaction is usually carried out in the presence of a hydrocarbon diluent such as heptane or a mixture of α-olefins. Less diluent is needed to obtain a stable reaction compared with chain growth using triethylaluminum as the feed. The reaction conditions, including residence time, are selected to add an average of from 0.50 to 3.0 and preferably from 0.75 to 2.0, and more preferably from 0.75 to 1.50, ethylene units to each R group (X_{T} = 0.75 to 1.50). Residence times of 10 to 120 minutes and, preferably, 30 to 90 minutes are suitable to peak the product at the desired carbon number, or numbers, and longer residence times, which cause the production of excessive amounts of higher olefins (X_{T} = 2 or more) should be avoided. The optimum conditions to maximize the amount of desired product (X_{T} = 1) can be readily determined for each system by following the teachings and working examples contained herein.

The product from the chain growth reaction is displaced with linear alpha-olefin containing 2, preferably 3 or more, and most preferably 4 to 10 carbon atoms to provide the product alpha-olefins and to regenerate trialkylaluminum which can be recycled to the chain growth reaction. Also, in order to minimize isomerization and dimerization of the product, a displacement catalyst is used which permits the process to be efficiently carried out at much lower temperatures (-20 to 200°C) compared to thermal displacement which uses temperatures of 300°C or more. The displacement process can be batch or continuous. A suitable process is described for example, in U.S. patent 4,918,254. The displacement can be carried out in a variety of reactor configurations. In a particularly advantageous embodiment, back-displacement is carried out continuously in a plug flow tubular or packed column reactor with the product alpha-olefins flashed from the reaction mixture at low temperatures in order to minimize isomerization.

Preferred catalysts are those which have the least isomerization activity under the conditions used and include, for example, cobalt carboxylates such as cobalt naphthenate. Nickel complexes, for example, nickel acetylacetonate, nickel carboxylates such as nickel naphthenate, nickel octanoate and nickel acetate, are suitable if used in combination with the addition of a catalyst poison to prevent isomerization. Although the cobalt catalysts are much less active for isomerization than the nickel catalysts, they are preferably also used in connection with a catalyst poison. Cyclodienes and acetylene hydrocarbons, such as phenylacetylene, can be used in the displacement reaction to suppress isomerization activity and prolong catalyst life. Effective amounts of catalyst depend upon the catalyst used. Generally, amounts of from 1 to 100 parts per million based on the weight of the reaction mixture can be used and, preferably 5-50 ppm. Reaction temperatures of from -20° to 200°C and preferably -10 to 100°C are suitable for catalyzed displacement. The aluminum alkyl feed to be displaced can be treated with an alpha-olefin to remove any aluminum hydride so as to extend catalyst life.

The amount of alpha-olefin fed to the displacement reaction should be in stoichiometric excess over the amount required to replace all alkyl groups. Preferably the amount of alpha-olefin should be at least a 200 percent excess over the stoichiometric amount required to replace all alkyl groups. Still more preferably the alpha-olefin feed should be at least a 500 percent stoichiometric excess over the trialkyl aluminum feed stream. In this manner, since the displacement reaction is an equilibrium reaction, the alkyl substitution in the trialkylaluminum product will more closely approach the distribution of the alpha-olefin feed.

Both displacement and side reactions (e.g., isomerization, dimerization, chain growth) proceed concurrently. However, the displacement reaction rate is much higher than the rate of the side reactions. This permits termination of the displacement reaction after a time that allows it to go substantially toward the equilibrium conversion and before a time in which the side reactions, especially isomerization, become significant. By "significant" is meant the amount of undesired by-products which would render the olefin effluent stream unsuitable for its intended purpose. The process is capable of making alpha-olefin product that is over 97 weight percent alpha-olefin based on tri-n-alkylaluminum converted.

Since all rates vary with temperature and amount of catalyst, the optimum time for termination under each specific condition will require a minimal amount of experimentation. In general when operating at 25°C, the reaction should be terminated after a reaction period of 30 seconds to 1 hour. A preferred reaction time is 1-20 minutes and most preferred 2-10 minutes. At higher temperatures, e.g., 50-100°C, the preferred reaction time before side reactions become significant will be shorter.

When the displacement has proceeded to the desired extent, usually close to reaction equilibrium, a catalyst poison is added in an amount that will deactivate the catalyst and prevent undesirable side reactions. These poisons include lead and copper and compounds thereof. Suitable lead compounds include, e.g., lead naphthenate, lead acetylacetonate, lead 2-ethylhexanoate, and tetraethyl lead. Suitable copper compounds include, e.g., copper naphthenate, copper acetylacetonate, cuprous bromide, and cuprous 2-ethylhexanoate. Use of the metals as the catalyst poison requires the metals to be in very finely divided forms and requires a greater amount of the catalyst poison. For example, amorphous lead metal is an effective catalyst poison at a Pb/Ni atom ratio of about 500. The catalyst poisons which are effective at the lowest concentrations have been lead compounds, e.g., lead naphthenate, lead 2-ethylhexanoate and lead acetylacetonate. The amount of catalyst poison should be an amount that effectively inhibits all undesired side reactions. With lead compounds a lead/nickel atom ratio of 1.0 has been effective and even lower amounts may be effective. Hence a useful Pb/Ni atom ratio is 0.5/1.0 to 5.0/1.0.

After the catalyst poison has been added, the trialkylaluminum product can be recovered by conventional methods such as distillation. When lead compounds are used as the poison, nickel and at least part of the lead form a precipitate which can be removed by filtration. We have found that the presence of residual Ni-Pb in trialkylaluminum recycled to the chain growth reaction does not appear to have any adverse effect on the reaction. Because the trialkylaluminum product also contains heavy olefins, a purge stream can be removed and subjected to catalytic displacement, such as with ethylene, to recover aluminum values as triethylaluminum which can then be fed to the chain growth reaction in order to improve the economics of the process.

The invention is further illustrated by, but is not intended to be limited to, the following Examples.

### Example 1A:

Displacement of tri-n-propylaluminum (TNPA) with 1-butene to yield tri-n-butylaluminum (TNBA) via nickel catalysis followed by poisoning of the nickel catalyst with lead, thermal conglomeration of the lead/nickel, filtration of the lead/nickel away from the TNBA, and finally ethylene chain growth of the TNBA solution.

### Procedure:

TNPA was pre-treated with 1-hexene at 60-70° C for about two hours to remove hydride impurity and excess 1-hexene was then vacuum-stripped off. 24 g of treated TNPA was charged into a 300 cc jacketed glass flask in the dry box. The flask, equipped with a thermocouple, a magnetic stir bar, a rubber septum and a refluxing condenser, was set up in a hood under a nitrogen pad. The reactor was cooled with a cooling circulator to about -15°C (to allow the reaction taking place at about atmospheric pressure) and ∼110 cc of pre-cooled 1-butene liquid was fed through a syringe needle (inserted into the septum) into the flask. 0.5 cc of nickel naphthenate in heptane solution (0.0056 g of Ni(II)/cc) was fed with a syringe to the reactor to catalyze the reaction. Samples were taken during the reaction to monitor the conversion. At the end of the reaction, 0.5 cc of lead carboxylate in heptane solution (0.029 g of Pb(II)/cc) was fed to kill the catalyst. The reactor was heated up to ∼120°C to drive off excess 1-butene and to conglomerate Pb/Ni. After the reaction was cooled down, ∼40 g. of heptane was added. Pb/Ni was filtered off in the dry box with a 30 cc syringe and a 0.5 micron PTFE filter.

### Analysis:

24 g TNPA starting, 70 g 1-C₄⁼, 2.8 mg Ni(II), -15°C, 120 minutes, 14.5 mg Pb (II). Results: Normalized % paraffin from hydrolysis of aluminum alkyl: C₃:16.1 C₄:73.8 C₆:10.1

### Examples 1B, 1C and 1D:

Example 1B represents the chain growth of TNBA resulting from displacement of TNPA in Example 1A and is compared to the chain growth of virgin TNBA (Example 1C) and to the chain growth of virgin TNBA spiked with lead plus nickel to show that the nickel catalyst is truly inert towards chain growth (Example 1D). Results were normalized to include even carbon number alkyls only (to eliminate the confusion from the partial amount of TNPA in Example 1B).

### Procedure:

### Chain Growth

A passivated 300 cc s.s. autoclave unit was set up and equipped with an electrical heating mantle, an internal coil with cooling water, a temperature indicator and controller, a safety disk, a magnetically coupled mechanical agitator, a pressure indicator, a 0.3 cm (1/8") dipleg connected to a ∼4.6m x 0.3 cm (15' x 1/8") s.s. sampling line and an ethylene feed line, a 150 cm³ s.s. feed funnel and a vent line. The reactor was pressure-tested and purged with nitrogen.

About 10 g of AlR₃ (e.g., TNBA with or without Pb/Ni fine particles) and 40 g of heptane (as the diluent) were charged into the 150 cc feed funnel in a dry box. The solution (or its mixture) was re-charged from the funnel to the autoclave under a N₂ pad. The reactor was pressure-tested and purged with ∼1.4 MPa (∼200 psig) ethylene twice.

After an initial pressurization with about 5.5 MPa (800 psig) ethylene, the reactor was heated up to a reaction temperature of 125°C (the set point) with good agitation. Ethylene was added during the reaction to maintain a pressure of 17.2-20.7 MPa (2500-3000 psig). After three pre-sampling purges for each sample, samples were taken periodically, with safety protection equipment, through the dipleg and the sampling line into a sample bottle containing cold aqueous HCl solution and heptane solvent.

After the reaction, the reactor was cooled down and excess ethylene was vented. The reaction solution could be stirred in the autoclave, if desired, to further passivate the reactor. Otherwise, the AlR₃ solution was discharged through the sampling line and neutralized with butanol solution in a bottle cooled with a water/ice bath. (Since the AlR₃ was so dilute, no safety problem was expected or encountered.) The autoclave was then washed four times with ∼140 cm³ of heptane, which was fed through the feed funnel. The last wash was left in the reactor until the next run. Samples were analyzed by GC with a 60m x 0.32mm SPB-1 capillary column. The GC analysis results, not including the heavier material above C₁₂, are reported in Table I. It can be seen that there is little C₄ olefin in the product and that short times are needed to peak the product at C₆. Under similar reaction conditions, but using a triethylaluminum feed, the amounts of less desirable C₄ in the product would be comparable to the amounts of the more valuable C₆ produced according to the process of the invention.

**TABLE I**

| % Paraffins by G.C. from Hydrolysis of Chain Growth Al Alkyl Products | | | | | |
|---|---|---|---|---|---|
| Sample Time (min.) | C₄ | C₆ | C₈ | C₁₀ | C₁₂ |
| | | | | | |

| Example 1B - TNBA | | | | | |
|---|---|---|---|---|---|
| 30 | 8.9 | 28.1 | 24.8 | 23.4 | 14.8 |
| 60 | 4.4 | 16.3 | 24.0 | 28.4 | 27.0 |

| Example 1C | | | | | |
|---|---|---|---|---|---|
| 30 | 11.5 | 31.1 | 22.5 | 19.8 | 15.1 |
| 60 | 6.6 | 21.4 | 22.5 | 25.1 | 24.5 |

| Example 1D | | | | | |
|---|---|---|---|---|---|
| 30 | 9.6 | 30.7 | 25.8 | 21.7 | 12.2 |
| 60 | 4.6 | 17.0 | 24.5 | 26.8 | 27.1 |

### Example 2

TNBA was partially displaced with 1-octene to yield a mixture of TNBA plus tri-n-octylaluminum (TNOA) followed by chain growth according to the procedure of Example 1B to yield an alkyl distribution enriched at C₆ and C₁₀. Displacement in this case is catalyzed by Co(II) instead of Ni(II); Pb is added at the end of the partial displacement. The cobalt is thermally agglomerated with the lead and then filtered prior to chain growth.

### Procedure:

The partial displacement reaction was carried out using 10 grams of TNBA and 5.6 grams of 1-octene and 1 mg of Co(II) catalyst at a temperature of 60°C and a time of 20 minutes. The catalyst was killed after 20 minutes by adding 5.3 mg of Pb (II). The normalized alkyl area % obtained after alkyl hydrolysis of the product aluminum alkyl showed 58% C₄ and 42% C₈. The chain growth reaction temperature was 125° C and the ethylene pressure 17.2-20.7 MPa (2500-3000 psig). The analysis of the chain growth product is given in Table II.

**TABLE II**

| % Paraffin from Al Alkyl Hydrolysis | | | | | | |
|---|---|---|---|---|---|---|
| Sample Time (min.) | C₄ | C₆ | C₈ | C₁₀ | C₁₂ | C₁₄ |
| 30 | 7.1 | 20.4 | 28.0 | 25.5 | 19.1 | -- |
| 60 | 2.9 | 8.7 | 18.4 | 22.8 | 24.9 | 22.2 |

## Claims

1. A process for making a linear alpha-olefin product which is enriched in one or more olefins of a selected carbon number ranging from 5 to 12 which process comprises:
(a) feeding ethylene and trialkylaluminum to a chain growth reaction zone, said trialkylaluminum consisting essentially of one or more compounds represented by the formula AlR₃, in which a major portion of the R groups, which can be the same or different, contain a number of carbon atoms which is two less than a selected carbon number,
(b) reacting said ethylene and said trialkylaluminum compound in said reaction zone so as to form a chain growth product in which the chain length of the R groups is increased by an average amount such that said product is enriched in alkyl groups having the selected carbon number,
(c) feeding said chain growth product, a displacement catalyst and displacing linear alpha-olefin, a major portion of which linear alpha-olefin contains a number of carbon atoms which is two less than said selected carbon number, to a displacement zone so as to form said linear alpha-olefin product and trialkylaluminum displacement product,
(d) after the displacement reaction has proceeded to the desired extent but before any significant isomerization of the linear alpha-olefin has occurred, deactivating said displacement catalyst with a deactivating amount of a catalyst poison selected from lead, copper and compounds thereof, and
(e) recycling said trialkylaluminum displacement product to said chain growth reaction zone.

2. The process of claim 1 wherein the chain growth reaction zone is at a temperature of from 100° to 150° C and a pressure of from 6.9-34.5 MPa (1000 to 5000 psig) and the chain length of the R groups is increased an average of from 0.50 to 3.0 ethylene units.

3. The process of claim 2 wherein the R groups in AlR₃ have from 3 to 10 carbon atoms.

4. The process of claim 1 wherein the chain length of the groups in said chain growth product is increased an average of from 0.75 to 1.50 ethylene units.

5. The process of claim 1 wherein said displacement catalyst is selected from nickel and cobalt compounds.

6. The process of claim 1 wherein the catalyst and catalyst poison are separated from the linear alpha-olefin product and trialkylaluminum by filtration.

7. The process of claim 5 wherein the displacement zone is at a temperature of from -20 to 200° C and said displacement catalyst is present in an amount of from 1 to 100 parts per million based on the total weight of the displacement reaction mixture.

8. The process of claim 1 wherein a heavy olefin containing purge stream is removed from said trialkylaluminum displacement product and subjected to a catalytic displacement to recover aluminum values as trialkylaluminum which is recycled to the chain growth reaction zone.

9. The process of claim 8 wherein said catalytic displacement to recover aluminum values is an ethylene displacement.

10. The process of claim 5 wherein the displacement catalyst is added as a nickel (II) or cobalt (II) compound.

11. The process of any of the foregoing claims wherein the catalyst poison is added as a Pb (II) compound.

## Patentansprüche

1. Verfahren zur Herstellung eines linearen α-Olefinprodukts, das mit einem oder mehreren Olefinen mit einer ausgewählten Kohlenstoffzahl im Bereich von 5 bis 12 angereichert ist, bei dem man
(a) Ethylen und Trialkylaluminium in eine Kettenwachstumszone einspeist, wobei das Trialkylaluminium im wesentlichen aus einer oder mehreren Verbindungen der Formel AlR₃ besteht, in der ein größerer Teil der R-Gruppen, die gleich oder verschieden sein können, eine Anzahl von Kohlenstoffatomen enthält, die um zwei unter einer ausgewählten Kohlenstoffzahl liegt;
(b) das Ethylen und die Trialkylaluminiumverbindung in der Reaktionszone zur Umsetzung bringt, um ein Kettenwachstumsprodukt herzustellen, in dem die Kettenlänge der R-Gruppen um eine durchschnittliche Menge verlängert ist, so daß das Produkt mit Alkylgruppen mit der ausgewählten Kohlenstoffzahl angereichert ist.
(c) das Kettenwachstumsprodukt, einen Verdrängungskatalysator und ein verdrängendes lineares α-Olefin, dessen überwiegender Teil eine Anzahl von Kohlenstoffatomen, die um zwei unter der gewählten Kohlenstoffzahl liegt, enthält, in eine Verdrängungszone einspeist, um das lineare α-Olefinprodukt und das Trialkylaluminium-Verdrängungsprodukt herzustellen;
(d) den Verdrängungskatalysator nach Ablauf der Verdrängungsreaktion bis zum erwünschten Ausmaß, doch vor Einsetzen einer erheblichen Isomerisierung des linearen α-Olefins, mit einer desaktivierenden Menge eines aus Blei, Kupfer und deren Verbindungen ausgewählten Katalysatorgifts desaktiviert und
(d) das Trialkylaluminium-Verdrängungsprodukt wieder in die Kettenwachstumszone zurückführt.

2. Verfahren nach Anspruch 1, bei dem die Kettenwachstumszone eine Temperatur von 100 bis 150°C und einen Druck von 6,9 bis 34,5 MPa (1000 bis 5000 psig) aufweist und die Kettenlänge der R-Gruppen um durchschnittlich 0,50 bis 3,0 Ethyleneinheiten zunimmt.

3. Verfahren nach Anspruch 2, bei dem die R-Gruppen in AlR₃ 3 bis 10 Kohlenstoffatome aufweisen.

4. Verfahren nach Anspruch 1, bei dem die Kettenlänge der Gruppen im Kettenwachstumsprodukt um durchschnittlich 0,75 bis 1,50 Ethyleneinheiten zunimmt.

5. Verfahren nach Anspruch 1, bei dem der Verdrängungskatalysator aus Nickel- und Cobaltverbindungen ausgewählt ist.

6. Verfahren nach Anspruch 1, bei dem der Katalysator und das Katalysatorgift durch Filtration vom linearen α-Olefinprodukt und Trialkylaluminium abgetrennt werden.

7. Verfahren nach Anspruch 5, bei dem die Verdrängungszone eine Temperatur von -20 bis 200°C aufweist und der Verdrängungskatalysator bezogen auf das Gesamtgewicht der Verdrängungsreaktionsmischung in einer Menge von 1 bis 100 ppm vorhanden ist.

8. Verfahren nach Anspruch 1, bei dem ein ein schweres Olefin enthaltender Spülstrom aus dem Trialkylaluminium-Verdrängungsprodukt entfernt und einer katalytischen Verdrängung unterzogen wird, um Aluminiumwerte als Trialkylaluminium zurückzugewinnen, das dann zur Kettenwachstumsreaktionszone zurückgeführt wird.

9. Verfahren nach Anspruch 8, bei dem die katalytische Verdrängung zur Rückgewinnung von Aluminiumwerten eine Ethylenverdrängung ist.

10. Verfahren nach Anspruch 5, bei dem der Verdrängungskatalysator als Nickel(II)- oder Cobalt(II)-verbindung zugesetzt wird.

11. Verfahren nach einem der vorstehenden Ansprüche, bei dem das Katalysatorgift als Pb(II)-verbindung zugesetzt wird.

## Revendications

1. Procédé pour fabriquer un produit d'alpha-oléfine qui est enrichi en une ou plusieurs oléfines présentant un nombre d'atomes de carbone sélectionné situé dans l'intervalle de 5 à 12, lequel procédé consiste à:
(a) introduire de l'éthylène et du trialkylaluminium dans une zone de réaction de croissance de chaîne, ledit trialkylaluminium étant constitué essentiellement d'un ou plusieurs composés représentés par la formule AlR₃, dans laquelle une majeure partie des groupes R, qui peuvent être identiques ou différents, contient un nombre d'atomes de carbone qui est deux fois inférieur à un nombre d'atomes de carbone sélectionné,
(b) faire réagir ledit éthylène et ledit composé de trialkylaluminium dans ladite zone de réaction de manière à former un produit de croissance de chaîne dans lequel la longueur de la chaîne des groupes R augmente en quantité moyenne, de manière à ce que ledit produit soit enrichi en groupes alkyle présentant le nombre d'atomes de carbone sélectionné,
(c) introduire ledit produit de croissance de chaîne, un catalyseur de déplacement et une alpha-oléfine linéaire de déplacement, une majeure partie de cette alpha-oléfine linéaire contenant un nombre d'atomes de carbone qui est deux fois inférieur audit nombre d'atomes de carbone sélectionné, dans une zone de déplacement, de manière à former ledit produit d'alpha-oléfine linéaire et ledit produit de déplacement de trialkylaluminium,
(d) désactiver ledit catalyseur de déplacement au moyen d'une quantité de désactivation d'un poison de catalyseur choisi parmi le plomb, le cuivre et des composés de ceux-ci, après que la réaction de déplacement ait progressé dans la mesure souhaitée, mais avant que toute isomérisation significative de l'alpha-oléfine linéaire se soit produite, et
(e) recycler ledit produit de déplacement de trialkylaluminium dans ladite zone de réaction de croissance de chaîne.

2. Procédé selon la revendication 1, dans lequel la zone de réaction de croissance de chaîne est à une température de 100° à 150°C et à une pression de 6,9 à 34,5 MPa (1.000 à 5.000 psig), et la longueur de chaîne des groupes R est augmentée en moyenne de 0,50 à 3,0 unités d'éthylène.

3. Procédé selon la revendication 2, dans lequel les groupes R dans AlR₃ présentent de 3 à 10 atomes de carbone.

4. Procédé selon la revendication 1, dans lequel la longueur de chaîne des groupes dans ledit produit de croissance de chaîne est augmentée en moyenne de 0,75 à 1,50 unités éthylène.

5. Procédé selon la revendication 1, dans lequel ledit catalyseur de déplacement est choisi parmi des composés de nickel et de cobalt.

6. Procédé selon la revendication 1, dans lequel le catalyseur et le poison du catalyseur sont séparés par filtration du produit d'alpha-oléfine linéaire et du trialkylaluminium.

7. Procédé selon la revendication 5, dans lequel la zone de déplacement est à une température de -20 à 200°C et ledit catalyseur de déplacement est présent en quantité de 1 à 100 parties par million, calculées sur base du poids total du mélange de réaction de déplacement.

8. Procédé selon la revendication 1, dans lequel on retire un courant de purge contenant des oléfines lourdes dudit produit de déplacement de trialkylaluminium, et on le soumet à un déplacement catalytique pour récupérer de l'aluminium sous forme de trialkylaluminium qui est recyclé dans la zone de réaction de croissance de chaîne.

9. Procédé selon la revendication 8, dans lequel ledit déplacement catalytique pour récupérer de l'aluminium est un déplacement par l'éthylène.

10. Procédé selon la revendication 5, dans lequel le catalyseur de déplacement est ajouté sous forme d'un composé de nickel (II) ou de cobalt (II).

11. Procédé selon l'une quelconque des revendications précédentes, dans lequel le poison du catalyseur est ajouté sous forme d'un composé de Pb (II).
